# EUROPÄISCHE PATENTSCHRIFT

(11) **EP 1 723 973 B1**
(45) Veröffentlichungstag und Bekanntmachung des Hinweises auf die Patenterteilung: **24.12.2014**
(21) Anmeldenummer: 06114162.8
(22) Anmeldetag: 18.05.2006
(51) Int. Cl.: A61L 9/12, A61L 9/04, A61L 9/16, A47C 31/00, A61L 9/18

(54) **Duftstofffreisetzende Möbel, Möbelteile und/oder Möbelzubehörteile**
Fragrance-releasing furnitures, furniture parts and/or furniture components
Meubles et parts et composantes de meubles à libération de parfums.

(30) Priorität: 18.05.2005 DE 102005023611
(43) Veröffentlichungstag der Anmeldung: 22.11.2006
(73) Patentinhaber: Fritz Egger GmbH & Co. OG, 3105 Unterradlberg (AT)
(72) Erfinder: Steinwender, Martin, Dr., A-2380 Perchtoldsdorf (AT)
(74) Vertreter: Cohausz & Florack

(56) Entgegenhaltungen:
- WO-A-99/05229
- DE-A1- 10 114 509
- DE-U1- 29 904 040
- US-A- 4 869 407
- US-A- 5 355 551
- PATENT ABSTRACTS OF JAPAN Bd. 2000, Nr. 03, 30. März 2000 (2000-03-30) & JP 11 348007 A (MATSUSHITA ELECTRIC IND CO LTD), 21. Dezember 1999 (1999-12-21)

## Beschreibung

Die Erfindung betrifft ein Möbelzubehörteil in Form eines Möbelbeschlags für Ladenauszüge oder eines Scharniers..

Im Zuge der industriellen Fertigung von Möbeln kommen heute in der Regel verschiedene Arten von Plattenwerkstoffen zur Anwendung. Neben Metall- und Kunststoffmaterialien haben sich im Möbel- und Innenausbau vor allem die verschiedenen Holzwerkstoffe besonders etabliert. Diese bieten aufgrund ihrer günstigen Eigenschaften hinsichtlich Gewicht, Festigkeit, einfacher Bearbeitbarkeit und Recyclierbarkeit sehr wirtschaftliche Bedingungen. Holzwerkstoffe bieten weiter die Möglichkeit mit einem Dekor bedruckt und lackiert zu werden oder mit einem dekorativen Schichtstoff versehen zu werden, womit im Wege des Plattenmaterials alle erdenklichen optischen Wirkungen erhalten werden können.

Dieser Umstand wird seit vielen Jahren genutzt um natürlich vorkommende Materialien zu imitieren. So sind es oft verschiedene Holzdekore mit denen der natürlich vorkommende Werkstoff Holz, der von je her eine bedeutende Rolle im Möbelbau gespielt hat, imitiert werden soll. Aber auch andere Gestaltungsmöglichkeiten von der gezielten Farbgebung in verschiedenem Glanzgrad über Fantasiedekore bis zu individuell gewählten Motiven können heute an Möbeloberflächen realisiert werden.

Neben der optischen Gestaltung wird auch durch strukturierte Oberflächen versucht, bestimmte haptische Effekte zu erzielen. Dies kann eine einfache, regelmäßige Rauhigkeit sein, die ihrerseits einen bestimmten Glanzgrad oder eine verringerte Fleckenempfindlichkeit bewirkt oder es können bestimmte Strukturen nachgebildet werden. Für letzteres kommen beispielsweise Porenstrukturen, wie sie die meisten Laubhölzer zeigen, oder Steinstrukturen, wie sie unterschiedlich bearbeitete Natursteinflächen zeigen, in Frage. Sind Struktur und Dekor aufeinander abgestimmt, stellt dies eine besonders gut wirkende Art der Nachbildung natürlich vorkommender Materialien dar.

Neben diesen optischen und haptischen Maßnahmen, mit denen Seh- und Tastsinn der Nutzer angesprochen werden sollen, sind aus dem Stand der Technik Elemente des Möbel- und Innenausbaues bekannt, die durch eine gezielte Freisetzung bestimmter Gerüche, eine weitere Form der Wahrnehmung ermöglichen. So ist es etwa aus der DE 103 32 000 A1 bekannt in einen Fußbodenbelag Duftstoffe einzubringen, wobei hier Mikrokapseln die Duftstoffe enthalten im oder auf dem Belag vorgesehen werden, die durch feuchtes Wischen gelöst werden um deren Geruchsstoffe freizusetzen. Weiteres können die Mikrokapseln in den Beschichtungsstoffen oder an den Kanten der Paneele vorgesehen sein und durch mechanische Einwirkung deren Inhalt freigeben.

Für Möbel ist es bekannt, durch Duftstoffe, die in das Plattenmaterial eingearbeitet werden, einen gewünschten Geruch oder antibakterielle oder insektiziede Wirkungen zu ermöglichen. So gibt etwa JP 11348007 A eine Holzwerkstoffplatte an, die einen Stoff enthält der einen Geruch absondert.

Diesen genannten Ausführungsformen haften jedoch verschiedene Nachteile an. So kann ein dosiertes abgeben von Duftstoffen, wenn diese in einer Platte enthalten sind nur sehr schwer realisiert werden. Möbel mit großen Flächen in Verhältnis zu deren Volumen bzw. Platzbedarf, werden ein sehr intensives Geruchspotential aufweisen. Hingegen wird der Effekt der Geruchsabgabe bei Möbeln aus gleichen Platten mit kompakter Bauweise - also geringem Plattenmaterial bezogen auf deren umbautes Volumen - vergleichsweise gering ausfallen. Zu dem besteht bei der Abgabe von Gerüchen aus dem Plattenmaterial selbst der Nachteil, dass diese Abgabe im Lauf der Zeit abnehmen wird. Die Wirkung von Mikrokapseln, wie sie aus dem Stand der Technik bei Fußbodenpaneelen bekannt ist, kann hier sinnvollerweise nicht angewendet werden, da die beschriebenen Mechanismen zur Freigabe deren Inhaltes wie Feuchte- oder mechanische Einwirkung nicht zur Verfügung stehen. Weiteres haftet jeder Art von Stoff, der über Sichtflächen abgegeben wird der Nachteil an, dass bei Hautkontakt allergische Reaktionen ausgelöst werden könnten.

Die DE 191 14 509 A offenbart die Anordnung von verkapselten Duftstoffen auf Möbeloberflächen, insbesondere auf Tischen oder Stühlen. Durch eine thermische Beanspruchung oder durch den Abrieb an Gegenständen im Alltagsgebrauch (z.B. durch Putzen oder Reibung) kann der Duftstoff freigesetzt werden.

Aus der US 5,355,551 ist die Anordnung von Duftstoffelementen an der Innenseite von Vorhangringen bekannt.

Aufgabe der vorliegenden Erfindung ist es daher eine Möglichkeit anzugeben, mit der sich Möbel herstellen lassen, die einen Duftstoff abgeben ohne die vorbeschriebenen Nachteile aufzuweisen.

Gelöst wird diese Aufgabe durch ein Möbelzubehörteil in Form eines Möbelbeschlags für Ladenauszüge oder eines Scharniers gemäß dem Patentanspruch 1 dadurch, dass die mindestens eine das Duftstoffelement aufweisende Oberflächen über die Öffnungs- und Schließmechanismen des Möbels bei dessen bestimmungsgemäßer Verwendung wiederkehrend mechanisch beansprucht wird, so dass das mindestens eine Duftstoffelement im Zuge des herkömmlichen Öffnens und/oder Schließens dosiert den Duftstoff freisetzt.

So ist erfindungsgemäß vorgesehen, Möbelbeschläge für Ladenauszüge mit Oberflächen vorzusehen, an denen - bei Betätigung der Lade - Abrollbewegungen von Wälzkörpern und/oder einfach mechanische Reibkräfte Duftstoffe freisetzen. Die Duftstoffe liegen vorzugsweise in verkapselter, insbesondere in mikroverkapselter Form vor. So kann an den bezeichneten Oberflächen insbesondere eine Matrix vorgesehen sein, die eine Vielzahl von Duftstoff enthaltene Kapseln (z.B. Mikrokapseln) aufweist, wobei jeweils oberflächennahe Kapseln geöffnet werden und deren Duftstoffe freigeben.

Erfindungsgemäß ist weiterhin vorgesehen, eine solche Fläche an einem Scharnier einer Schranktüre vorzusehen. Auch hier können Reibkräfte genutzt werden, bei Betätigung der Schranktüre Duftstoffe freizusetzen.

Auch ist es möglich, die mit verkapselten (insbesondere mikroverkapselten) Duftstoffen versehenen Oberflächen an Stellen vorzusehen, an denen bestimmungsgemäß Oberflächen aneinanderschlagen. Hier sei beispielsweise etwa eine Schranktüre erwähnt, die beim Schließen gegen einen Korpusteil schlägt oder ein Teil einer Lade der beim Schließen der Lade auf eine bestimmte Fläche des feststehenden Möbelteils trifft.

Offenbart werden weiterhin solche Zubehörteile, die im Zuge der Möbelherstellung in Form von Verbindungselementen Verwendung finden. So ist es auch vorgesehen, beispielsweise Dübel, wie sie für die verbesserte Verleimung von Korpusteilen vorgesehen sind, oder Gewindeschrauben mit einer Matrix Duftstoffe enthaltener Mikrokapseln zu versehen. Im Zuge der Möbelherstellung wird dann der Duftstoff in die Platte abgegeben, der von dort verzögert, und nur an vorgegebenen Stellen freigegeben wird. Eine verbesserte Form über Verbindungselemente Duftstoffe freizusetzen, ist diese an Flächen von Elementen vorzusehen, die im Zuge der Montage der Möbel mechanische Einwirkungen erfahren, am fertigen Möbel aber trotzdem mit der Umgebungsluft in Kontakt stehen. Dies wäre etwa der Fall bei Winkelverbindern aus Spannbolzen mit Exzenterschrauben.

Weiterhin offenbart werden sogenannte Faltplatten, bei denen Plattenteile über ein Klebstoffscharnier zusammen gehalten werden, wobei hier das Klebstoffscharnier die Matrix bilden kann und bei Auffalten der Platte durch die Verformungen im Klebstoffscharnier Duftstoff enthaltende Mikrokapseln deren Inhalt freigeben.

Daneben sind alle erdenklichen Öffnungs- und Schließmechanismen bei deren bestimmungsgemäßer Verwendung wiederkehrend Oberflächen miteinander in Kontakt treten, für die erfindungsgemäße Art der Beduftung verwendbar. So können neben den beispielhaft aufgeführten Möglichkeiten z. B. auch Oberflächen bei Karussellvorrichtungen, Rollmechanismen, Schiebetürelementen, etc. erfindungsgemäß ausgestatten werden.

Bei den Möbel- und Möbelzubehörteilen kann es sich beispielsweise um Bauteile handeln, aus denen ein Möbelstück zusammengefügt wird. Ganz allgemein können die Bauteile aus gleichen oder unterschiedlichen Materialien bestehen. Dabei kommt es nicht auf die Art des jeweiligen Materials an, es können Kunststoffe, Metalle und natürliche Materialien zum Einsatz kommen.

In bevorzugter Weise besteht dabei jeweils mindestens ein Bauteil zumindest teilweise aus einem zellulosehaltigen Material, beispielsweise aus einem zumindest teilweise aus einjährig nachwachsenden Pflanzen wie Gras oder Stroh bestehenden Material, das vorzugsweise verpresst und/oder mit einem Bindemittel versehen ist. Insbesondere kann das Material auch zumindest teilweise aus einem lignozellulosehaltigen Material bestehen, wie Holz oder einem Holzwerkstoff.

Erfindungsgemäß können die Duftstoffe in Kapseln, insbesondere in Mikrokapseln, eingebracht werden, aus denen sie dann bei Bedarf freigesetzt werden können. Mikrokapseln dieser Art sind grundsätzlich bekannt. Als Kapsel im Sinne dieser Erfindung zählt jede Art von Umhüllung eines Stofftröpfchens und/oder Stoffkorns.

Eine Vielzahl von so erhaltenen Kapseln können erfindungsgemäß in einer Matrix dispergiert sein und erfindungsgemäß eingesetzt werden. Die Matrix, in der die Kapseln dispergiert sind, hat primär die Aufgabe, die Kapseln auf der Oberfläche der Fläche so nachhaltig zu verankern, dass sie bis zum Zeitpunkt der Freisetzung des darin enthaltenen Stoffes an der Oberfläche verweilen. Zur Herstellung einer Matrix bestehend aus einer Vielzahl von Kapseln kann beispielsweise ein Wachs, Klebstoff und/oder Harz als Bindemittel eingesetzt werden.

Das Aufbrechen der Kapseln erfolgt erfindungsgemäß durch die Einwirkung von mechanischen Kräften, also beispielsweise durch Druck oder Reibung. Für die Freisetzung der in den Kapseln enthaltenen Stoffe, insbesondere der Duftstoffe, sind neben einer Druck- oder Reibungskrafteinwirkung aber auch alle anderen Techniken, die ein Aufbrechen der Kapseln bewirken, geeignet. Das Aufbrechen der Kapseln kann durch die Verwendung von Hilfsmitteln wie z. B. durch das Auftragen einer die Kapsel auflösenden Flüssigkeit, wie z. B. Wasser durch Sprühen, Streichen, Rollen oder ähnliches, durch die Einwirkung von Ultraschallenergie, Hochfrequenzenergie, Wärmeenergie z. B. Infrarot-Strahlung oder die Einwirkung von UV-Strahlung erfolgen.

Als Duftstoffe kommen jegliche feste oder flüssige Stoffe in Frage, die geeignet sind, einen Duftstoff freizusetzen. Hierbei handelt es sich beispielsweise um ätherische Öle. Besonders geeignet für den Einsatz in Kapseln sind auch Duftstoffe in Gelform. Erfindungsgemäß besonders geeignete Duftstoffe sind beispielsweise solche, die den Geruch von Holz freisetzen.

Die Erfindung soll jedoch nicht darauf beschränkt sein, Duftstoffe zu verwenden, die den Eigengeruch von Holz nachbilden. Vielmehr kommen auch Duftstoffe in Betracht, die generell als angenehm empfunden werden und aus einer großen Palette der auf dem Markt angebotenen Duftstoffe ausgewählt werden können. Es ist im übrigen auch bekannt, dass es Duftstoffe gibt, die bei den meisten Menschen angenehme Assoziationen oder Gefühle auslösen. Als angenehm empfunden werden im allgemeinen Orangen- oder Zitronendüfte.

Erfindungsgemäß besonders bevorzugte Duftstoffe sind solche, die den Geruch von Holz, Rosen, Blüten, Kräutern freisetzen. Mit derartigen Duftstoffen erfindungsgemäß ausgestattete Möbelzubehörteile können beispielsweise zur Innenausstattung im Ladenbau, z. B. für Möbel in Geschäfts- und Verkaufsräumen, oder auch für Möbel im Wohn- und/oder Schlafbereich eingesetzt werden.

Ein weiterer Einsatzbereich der Erfindung besteht darin, Duftstoffe in Möbel einzuarbeiten, die Ungeziefer vertreiben, von Menschen dagegen nicht als unangenehm empfunden werden. Zu letzteren zählen beispielsweise Duftstoffe gegen Motten.

Die Erfindung wird im Folgenden anhand spezieller Ausführungsbeispiele und der beigefügten Zeichnung näher erläutert. In der Zeichnung zeigen
- Fig. 1: einen Ausschnitt eines teilweise auf der Oberfläche eines Möbelelements angeordneten Duftstoffelements,
- Fig. 2: einen Ausschnitt mit einem in einer Matrix angeordneten verkapselten Duftstoff,
- Fig. 3: ein aus Möbelteilen bestehendes Möbel mit einem an dem Anschlag einer Tür angeordneten Duftstoffelement,
- Fig. 4: eine Schublade mit einem in einer seitlichen Schiene angeordneten Duftstoffelement,
- Fig. 5: eine Tischplatte mit einem in den Seitenkanten eingelassenen Duftstoffelement und
- Fig. 6: ein Scharnier mit zwischen den sich gegeneinander bewegenden Flächen der Gewerbe angeordneten Duftstoffelementen.

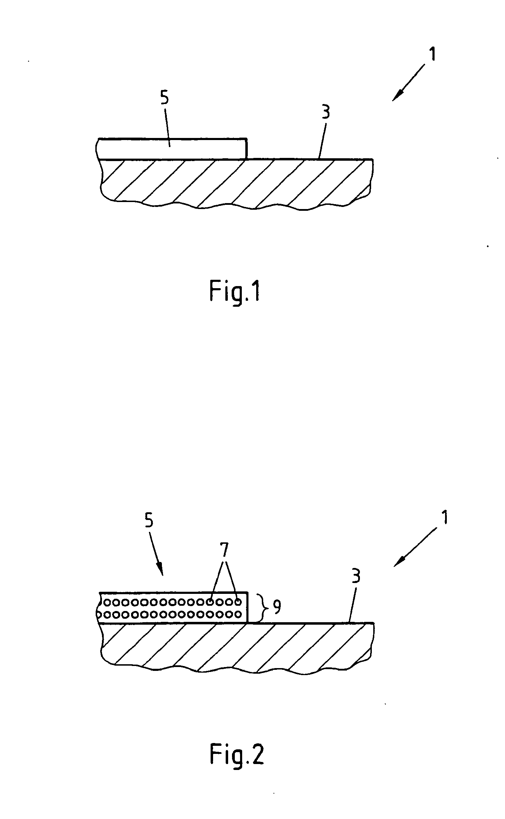

Fig. 1 zeigt ein Möbelelement 1 mit einer Oberfläche 3 und mit einem einen Duftstoff aufweisenden Duftstoffelement 5, wobei das Duftstoffelement 5 teilweise auf der Oberfläche 3 angeordnet ist. In dem gezeigten Ausschnitt kann das Duftstoffelement 5 so ausgeführt sein, dass es durch eine mechanische Beanspruchung und/oder durch eine durch Feuchtigkeit bewirkte Beanspruchung dosiert den Duftstoff freisetzt. Diese Varianten werden im Folgenden näher erläutert.

Weiterhin kann es sich um eine Beanspruchung in Form von Strahlung, beispielsweise Tageslicht, handeln, durch die dosiert der Duftstoff freigesetzt wird. Ist die Oberfläche 3 eine Außenseite eines Möbels, kann ein Duftstoff während des Tages freigesetzt werden. Alternativ kann die Oberfläche 3 eine Innenseite eines Möbelteils von einem Schrank sein, so dass beim Öffnen der Schranktür der Lichteinfall ein dosiertes Freisetzen des Duftstoffes bewirkt.

In Fig. 2 ist gezeigt, dass das Möbelelement 1 auf seiner Oberfläche 3 ein Duftstoffelement 5 aufweist, das eine mikroverkapselte Duftstoffe 7 aufweisende Matrix 9 aufweist. So kann das Duftstoffelement 5 in besonders einfacher Form hergestellt und auf der Oberfläche 3 angeordnet werden. Insbesondere kann es sich bei der Matrix um einen Wachs, Klebstoff und/oder Harz handeln. Durch entsprechende Ausgestaltung der Duftstoffkapseln 7 und der Matrix 9 können die Duftstoffkapseln 7 den Duftstoff bei Beanspruchung dosiert freisetzen.

Unabhängig davon, ob der Duftstoff in verkapselter Form vorgesehen ist, kann der Duftstoff aus einem festen, gelartigen oder flüssigen Stoff, insbesondere ätherischen Ölen hergestellt sein.

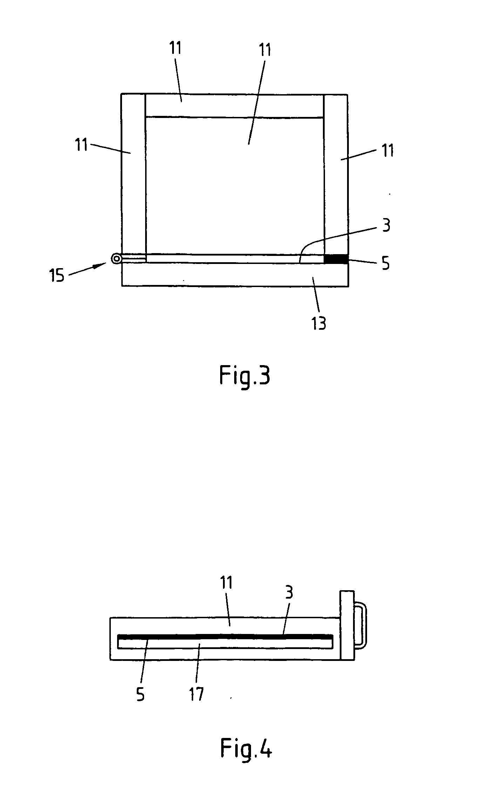

In Fig. 3 ist ein Möbel in Form eines Schrankes gezeigt, der aus mehreren Möbelteilen 11 zusammengebaut ist. Ein Möbelteil 11 ist eine Schranktür 13, die durch ein Scharnier 15 schwenkbar mit dem aus den Möbelteilen 11 gebildeten Korpus verbunden ist. Das Duftstoffelement 5 ist teilweise an der Oberfläche 3 der Schranktür 13 in einem Bereich angeordnet, der beim Schließen der Schranktür 13 an einem Möbelteil 11 anschlägt. Beim Öffnen der Schranktür 13 kann ebenfalls eine mechanische Beanspruchung, beispielsweise ein sich Ablösen vom Möbelelement 11, vorliegen. Durch diese mechanische Beanspruchung setzt das Duftstoffelement 5 bei jedem Schließen und ggf. Öffnen der Schranktür 13 dosiert Duftstoffe frei. Das Duftstoffelement 5 kann auf jeder beliebigen anderen Oberfläche 3 eines beliebigen Möbelteils 11, beispielsweise am Boden und/oder Haupt sowie an Einlegeböden, angeordnet werden, die mechanisch beansprucht werden.

In einer nicht gezeigten Ausführungsform kann die Schranktür 13 auch als Schiebetür ausgeführt sein, so dass beim Verschieben der Schranktür 13 durch entsprechend angebrachte Duftstoffelemente 5 dosiert Duftstoffe freigesetzt werden.

In dem in Fig. 4 gezeigten Ausführungsbeispiel einer Schublade ist das Duftstoffelement 5 an einer Oberfläche 3 einer Nutflanke einer Nut 17 angeordnet, die in einem Möbelteil 11 der Schublade vorgesehenen ist und zur Führung der Schublade dient. Im eingebauten Zustand der Schublade greifen Führungselemente in die Nut 17 ein. Beim Öffnen und Schließen der Schublade wird dann das Duftstoffelement 5 mechanisch durch Reibung beansprucht und setzt dosiert Duftstoffe frei.

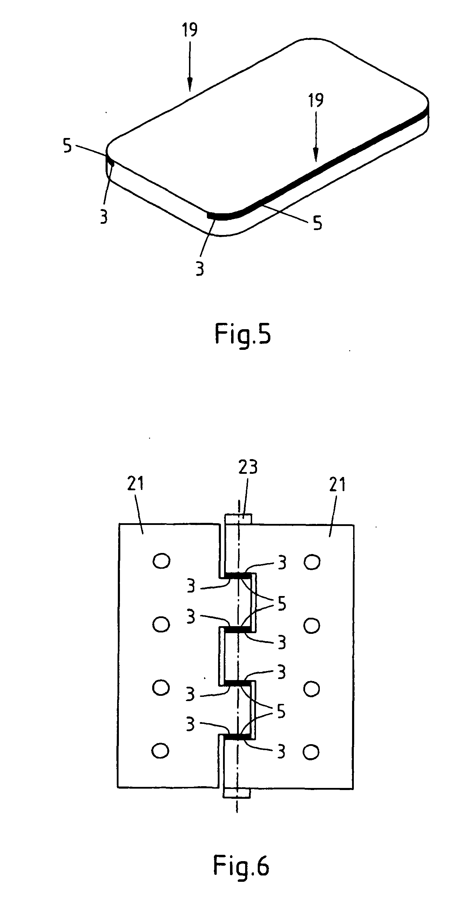

Fig. 5 zeigt eine Tischplatte, wobei an den Oberflächen 3 der Kanten 19 Duftstoffelemente 5 angeordnet sind. Die in Fig. 5 gezeigten Duftstoffelemente 5 können insbesondere so ausgestaltet sein, dass eine Beanspruchung durch Feuchtigkeit zu einem dosierten Freisetzen der Duftstoffe führt. Somit wird bei jeder Reinigung, beispielsweise mit einem feuchten Tuch, der Tischplatte ein Duftstoff dosiert freigesetzt.

Bei den bisher gezeigten Ausführungsbeispielen war die mindestens eine Oberfläche 3 jeweils an einem Möbelteil angeordnet.

Wie im Folgenden gezeigt, ist erfindungsgemäß die mindestens eine Oberfläche 3 an einem Möbelzubehörteil angeordnet. Ebenso kann auch eine Kombination aus Möbelteilen sowie Möbelzubehörteilen gewählt werden.

Fig. 6 zeigt ein Möbelzubehörteil in Form eines Scharniers. Dabei kann es sich um jede Art von Scharnier, beispielsweise einem Aushängeband, Flachband, Topfscharnier, Soss-Scharnier, Zysa-Scharnier, Winkelscharnier, Klappscharnier, Zapfenband und dergleichen oder, wie in Fig. 6 gezeigt, einem gerollten Scharnier handeln.

Das an sich bekannte Scharnier besteht aus zwei Scharnierelementen 21 die über einen genieteten Stift 23 drehbar miteinander verbunden sind. Auf den sich gegeneinander bewegenden Oberflächen 3 der Scharnierelemente 21 sind jeweils Duftstoffelemente 5 angeordnet. Bei Drehung der Scharnierelemente 21 gegeneinander werden die Duftstoffelemente 5 mechanisch durch Reibung beansprucht und setzen so dosiert Duftstoffe frei. Je nach Art des Scharniers können die Duftstoffelemente 5 auf verschiedenen Oberflächen 3 des Scharniers angeordnet werden.

Es kann sich bei dem Möbelzubehörteil auch um eine nicht gezeigte Schiene zur Führung beispielsweise einer Schublade oder einer Schiebetür handeln. Weiterhin können Möbelzubehörteile auch Rollen sein.

Die Erfindung ist nicht auf die zuvor gezeigten Ausführungsbeispiele beschränkt. Insbesondere ist jedes Möbelzubehörteil in Form eines Ladenauszugs oder eines Scharniers, mit den zuvor im allgemeinen und speziellen Teil beschriebenen Merkmalen Gegenstand dieser Erfindung.

## Patentansprüche

1. Möbelzubehörteil eines Möbels in Form eines Möbelbeschlags für Ladenauszüge oder eines Scharniers,
- mit mindestens einer Oberfläche (3) und
- mit mindestens einem einen Duftstoff aufweisenden Duftstoffelement (5), wobei das Duftstoffelement (5) mindestens teilweise auf der mindestens einen Oberfläche (3) angeordnet ist, wobei
- die mindestens eine das Duftstoffelement (5) aufweisende Oberfläche (3) über die Öffnungs- und Schließmechanismen des Möbels bei dessen
bestimmungsgemäßer Verwendung wiederkehrend mechanisch beanspruchbar ist, so dass das mindestens eine Duftstoffelement (5) im Zuge des herkömmlichen Öffnens und/oder Schließens dosiert den Duftstoff freisetzen kann.

2. Möbelzubehörteil nach Anspruch 1,
**dadurch gekennzeichnet,**
**dass** das mindestens eine Duftstoffelement (5) einen verkapselten, insbesondere mikroverkapselten Duftstoff (7) aufweist.

3. Möbelzubehörteil nach Anspruch 1 oder 2,
**dadurch gekennzeichnet,**
**dass** das mindestens eine Duftstoffelement (5) eine einen Duftstoff aufweisende Matrix (9) aufweist.

4. Möbelzubehörteil nach Anspruch 3,
**dadurch gekennzeichnet,**
**dass** die Matrix (9) aus einem Wachs, Klebstoff und/oder Harz hergestellt ist.

5. Möbelzubehörteil nach einem der Ansprüche 1 bis 4, **dadurch gekennzeichnet,**
**dass** der Duftstoff aus einem festen, gelartigen oder flüssigen Stoff, insbesondere ätherischen Ölen hergestellt ist.

6. Möbelzubehörteil nach einem der Ansprüche 1 bis 5, **dadurch gekennzeichnet,**
**dass** die mindestens eine Beanspruchung zusätzlich eine durch Feuchtigkeit bedingte Beanspruchung ist.

7. Möbelzubehörteil nach einem der Ansprüche 1 bis 6, **dadurch gekennzeichnet,**
**dass** die mindestens eine Beanspruchung zusätzlich eine durch Strahlung bedingte Beanspruchung ist.

8. Möbelzubehörteil nach einem der Ansprüche 1 bis 7, **dadurch gekennzeichnet,**
**dass** die mindestens eine Oberfläche (3) an einer Schublade angeordnet ist.

9. Möbelzubehörteil nach einem der Ansprüche 1 bis 8, **dadurch gekennzeichnet,**
**dass** die mindestens eine Oberfläche (3) an einem Scharnier angeordnet ist.

10. Möbelzubehörteil nach einem der Ansprüche 2 bis 9, **dadurch gekennzeichnet,**
**dass** die mindestens eine Oberfläche (3) an Stellen vorgesehen ist, an denen bestimmungsgemäß Oberflächen aneinander schlagen.

11. Möbel mit mindestens einem Möbelzubehörteil (1) nach einem der Ansprüche 1 bis 10.

## Claims

1. Furniture accessory of an item of furniture in the form of a furniture fitting for drawer pullouts or a hinge,
- having at least one surface (3) and
- having at least one scented substance element (5) having a scented substance, whereby the scented substance element (5) is at least partially arranged on the at least one surface (3),
whereby
- the at least one surface (3) with the scented substance element (5) can be repeatedly subjected to mechanical stress by means of the opening and closing mechanisms of the furniture when used as intended, such that the at least one scented substance element (5) can release the scent in doses during the normal opening and/or closing.

2. Furniture accessory according to Claim 1,
**characterised in that** the at least one scented substance element (5) has a scented substance (7) in capsules, in particular in microcapsules.

3. Furniture accessory according to either Claim 1 or Claim 2,
**characterised in that** the at least one scented substance element (5) has a matrix (9) containing a scented substance.

4. Furniture accessory according to Claim 3,
**characterised in that** the matrix (9) is made from a wax, adhesive and/or resin.

5. Furniture accessory according to any one of Claims 1 to 4,
**characterised in that** the scented substance is made of a solid, gel-like or liquid substance, in particular essential oils.

6. Furniture accessory according to any one of Claims 1 to 5,
**characterised in that** the at least one stress is additionally a stress caused by moisture.

7. Furniture accessory according to any one of Claims 1 to 6,
**characterised in that** the at least one stress is additionally a stress caused by radiation.

8. Furniture accessory according to any one of Claims 1 to 7,
**characterised in that** the at least one surface (3) is arranged on a drawer.

9. Furniture accessory according to any one of Claims 1 to 8,
**characterised in that** the at least one surface (3) is arranged on a hinge.

10. Furniture accessory according to any one of Claims 2 to 9,
**characterised in that** the at least one surface (3) is provided at locations at which during intended use surfaces impact one another.

11. Item of furniture having at least one furniture accessory (1) according to any one of Claims 1 to 10.

## Revendications

1. Accessoire de meuble en forme de ferrure pour tiroir ou de charnière,
- avec au moins une surface (3) et
- avec au moins un élément odoriférant (5) doté d'une matière odoriférante, l'élément odoriférant (5) étant disposé, au moins partiellement, sur la surface (3) au moins prévue, sachant que
- La surface (3) au moins prévue, dotée de l'élément odoriférant (5), peut être sollicitée mécaniquement, de manière récurrente, par le mécanisme d'ouverture et de fermeture du meuble, lors de son utilisation normale, de telle sorte que ledit élément odoriférant (5) au moins présent puisse libérer une dose de matière odoriférante lors de l'ouverture et / ou de la fermeture habituelles.

2. Accessoire de meuble selon la revendication 1,
**caractérisé en ce que** l'élément odoriférant (5) au moins prévu est doté d'une matière odoriférante encapsulée (7), en particulier micro-encapsulée.

3. Accessoire de meuble selon revendication 1 ou 2,
**caractérisé en ce que** l'élément odoriférant (5) au moins prévu est pourvu d'une matrice (9) dotée d'une matière odoriférante.

4. Accessoire de meuble selon la revendication 3,
**caractérisé en ce que** la matrice (9) est fabriquée en cire, en colle et / ou en résine.

5. Accessoire de meuble selon l'une des revendications 1 à 4, **caractérisé en ce que** la matière odoriférante est fabriquée à partir d'une matière solide, genre gel, ou liquide, en particulier à partir d'huiles éthérées.

6. Accessoire de meuble selon l'une des revendications 1 à 5, **caractérisé en ce que** l'au moins une sollicitation est de plus une sollicitation due à l'humidité.

7. Accessoire de meuble selon l'une des revendications 1 à 6, **caractérisé en ce que** l'au moins une sollicitation est de plus une sollicitation due à un rayonnement.

8. Accessoire de meuble selon l'une des revendications 1 à 7, **caractérisé en ce que** la surface (3) au moins prévue est disposée sur un tiroir.

9. Accessoire de meuble selon l'une des revendications 1 à 8, **caractérisé en ce que** la surface (3) au moins prévue est disposée sur une charnière.

10. Accessoire de meuble selon l'une des revendications 2 à 9, **caractérisé en ce que** la surface (3) au moins prévue est placée à des endroits auxquels des surfaces se heurtent conformément au but.

11. Meuble avec au moins un accessoire de meuble (1) selon l'une des revendications 1 à 10.
